# EUROPÄISCHE PATENTSCHRIFT

(11) **EP 2 670 903 B1**
(45) Veröffentlichungstag und Bekanntmachung des Hinweises auf die Patenterteilung: **01.07.2015**
(21) Anmeldenummer: 12707466.4
(22) Anmeldetag: 27.01.2012
(51) Int. Cl.: A45D 7/02, D06M 10/02, A41G 3/00, A61N 1/40, B01J 19/08, H05H 1/24, D06P 5/20, A61Q 5/00, A61N 1/32

(54) **VERFAHREN ZUR BEHANDLUNG VON MENSCHLICHEM ODER TIERISCHEM HAAR UND GERÄT ZUR DURCHFÜHRUNG DES VERFAHRENS**
METHOD FOR TREATING HUMAN OR ANIMAL HAIR AND APPARATUS FOR CARRYING OUT THE METHOD
PROCÉDÉ DE TRAITEMENT DE CHEVEUX HUMAINS OU DE POILS D'ANIMAUX ET APPAREIL PERMETTANT DE METTRE EN OEUVRE LE PROCÉDÉ

(30) Priorität: 02.02.2011 DE 102011010273
(43) Veröffentlichungstag der Anmeldung: 11.12.2013
(73) Patentinhaber: Cinogy GmbH, 37115 Duderstadt (DE)
(72) Erfinder: WANDKE, Dirk, 37308 Heilbad Heiligenstadt (DE); KOPP, Matthias, 37434 Gieboldehausen (DE); STORCK, Karl-Otto, 37115 Duderstadt (DE); SEGL, Maximilian, 37115 Duderstadt (DE); TRUTWIG, Leonhard, 37115 Duderstadt (DE); SCHARF, Johannes, 51467 Bergisch-Gladbach (DE)
(74) Vertreter: Lins, Edgar
(86) Internationale Anmeldenummer: PCT/DE2012/000091
(87) Internationale Veröffentlichungsnummer: WO 2012/103877

(56) Entgegenhaltungen:
- EP-A1- 0 592 979
- EP-A2- 1 367 172
- US-A- 5 743 278
- US-A1- 2010 212 683

## Beschreibung

Die Erfindung betrifft ein Verfahren zur Behandlung von auf einem Träger fest angeordnetem menschlichen oder tierischen Haar.

Die Erfindung betrifft ferner ein Gerät zur Durchführung des Verfahrens.

Es sind zahlreiche Behandlungen von Haaren bekannt, das sich fest an einem Träger angeordnet befindet. Der Träger kann dabei ein geeignetes Gewebe einer Perücke oder eines Haarteils, aber auch die menschliche oder tierische Haut sein. Wenn sich das haar an dem zugehörigen menschlichen oder tierischen Körper befindet und von dem Körper versorgt wird, kann es insbesondere Wachsen und seine Funktionsstruktur erhalten ("lebendes Haar"). Zu den Behandlungen des Haares gehören Waschbehandlungen, mit denen das Haar von Verunreinigungen befreit und ggf. desinfiziert wird. Es ist ferner bekannt, Haare mit chemischen Mitteln zu färben, für eine Färbung vorzubereiten oder in ihrer Struktur so zu verändern, dass sie eine gewünschte Form, beispielsweise Lockenform, annehmen. Derartige Verfahren können unter Anwendung von Hitze, aber auch bei Umgebungstemperatur mit dem Einsatz chemischer Mittel angewendet werden.

Durch EP 0 592 979 A1 ist es bekannt, die Oberfläche von tierischem Haar durch eine Plasmabehandlung zu modifizieren, um das Haar für eine anschließende Färbung geeigneter zu machen. Hierbei handelt es sich jedoch um abgeschnittene tierische Haare, die in einer Plasmakammer mit einem durch ein Plasma zu einer Glimmentladung anregbaren Gas behandelt werden. Eine derartige Behandlung ist naturgemäß für das noch an einem Träger fest angeordnete, insbesondere für das noch am Körper befindliche lebende Haar nicht geeignet.

Durch EP 1 367172 A2 ist eine Plasmabehandlung von Keratinfasern bekannt, durch die Keratinfasern zur Färbung vorbereitet und gegen Verfilzung geschützt werden. Auch hier findet die Behandlung in einer Plasmakammer statt.

Durch US 2010/0212683 A1 ist ein zangenartiges Gerät bekannt, mit dem Haarsträhnen erfasst und einer Haarbehandlung unterzogen werden. Für die Haarbehandlung ist die Zange mit einem Ultraschallwellengenerator und wenigstens einem Ionengenerator versehen. In dem Ionengenerator befindet sich eine Ionisierungselektrode, sodass auf die ergriffenen Haare in dem Ionengenerator erzeugte Ionen gegen die Haare der ergriffenen Haarsträhne geleitet werden können. Eine Plasmabehandlung ist nicht offenbart. Dementsprechend ist auch keine Hochspannungsversorgung an dem Behandlungsgerät vorgesehen.

US 5,743,278 offenbart ein kammähnliches Behandlungsgerät für Haare, mit dem eine elektrolytische Behandlung mit einer Behandlungsflüssigkeit ermöglicht ist. Dabei werden benachbarte Zinken des kammähnlichen Geräts mit unterschiedlichen Polen einer Gleichspannungsversorgung verbunden, um so für die Behandlungsflüssigkeit jeweils ein Element aus zwei unterschiedlichen Elektroden zu bilden.

Der vorliegenden Erfindung liegt die Aufgabe zugrunde, die Behandlung von auf einem Träger fest angeordnetem menschlichen oder tierischen Haar zur Verbesserung der Oberflächeneigenschaften, insbesondere zur Vorbereitung einer anschließenden chemischen Behandlung, zu ermöglichen.

Zur Lösung dieser Aufgabe ist erfindungsgemäß bei einem Verfahren der eingangs erwähnten Art vorgesehen, dass das Haar zu Strähnen aufgeteilt wird und dass die abgeteilten Strähnen durch ein Durchziehen eines mit einer Hochspannungsquelle verbundenen Geräts durch die Strähne einer dielektrischen Plasmabehandlung unterworfen werden.

Das erfindungsgemäße Verfahren ermöglicht die Behandlung von nicht abgeschnittenem menschlichen oder tierischen Haar durch eine dielektrisch behinderte Plasmabehandlung. Dadurch können die mit einer Plasmabehandlung erzielbaren Vorteile in einfacher Weise bei einem auf dem Träger fest angeordneten Haar, insbesondere bei lebendem Haar, erzielt werden. Diese Vorteile liegen insbesondere in der verstärkten Aufnahmefähigkeit des Haares, beispielsweise für einen Farbstoff, in einer desinfizierenden Wirkung usw.

In einer ersten Ausführungsform des erfindungsgemäßen Verfahrens wird die abgeteilte Strähne zwischen zwei zangenförmig angeordneten flächigen Elektroden erfasst, die zur Strähne hin mittels jeweils eines Dielektrikums abgedeckt sind. Die Elektroden werden mit einer Hochspannungsquelle verbunden, die insbesondere eine hochfrequente Wechselspannungsquelle ist. Zwischen den beiden Elektroden wird das strähnenförmig abgeteilte Haar eingeklemmt, indem die Hebel der Zange gegeneinander bewegt werden. In dem Zustand, in dem beide Hebel der Zange leicht gegeneinander gedrückt werden, wird die Haarsträhne der Länge nach durch das Gerät gezogen, wobei zwischen den Elektroden das Hochspannungsfeld vorhanden ist.

Durch die Dielektrika, die die Elektroden zur Strähne hin abdecken, wird der Stromfluss begrenzt, sodass beispielsweise eine Funkenentladung nicht stattfinden kann. Vielmehr wird im Zwischenraum zwischen den Dielektrika, in dem sich die Haare befinden, die dort vorhandene Luft ionisiert, sodass sich dort das Plasma ausbildet. Die Oberfläche der zwischen den mit den Dielektrika abgedeckten Elektroden hindurchgezogenen Haare wird durch das so gebildete Plasma modifiziert. Auf diese Weise kann beispielsweise das gesamte Haupthaar eines Menschen strähnenweise plasmabhehandelt werden.

In einer Ausführungsform des erfindungsgemäßen Verfahrens wird die Aufteilung des Haars in Strähnen mittels einer kammähnlichen Struktur mit parallel zueinander verlaufenden Zinken vorgenommen, wobei die Zinken mit einem leitenden, allseitig mit einem Dielektrikum umgebenden Kern ausgebildet sind und mit dem leitenden Kern mit einer Hochspannungsquelle verbunden werden. Die Zinken können dabei gemeinsam eine Elektrode der Hochspannungsquelle bilden, während der die Haare tragende Körper, vorzugsweise nahe dem behandelten Bereich der Haare, mit einem Referenzpotential, beispielsweise in Form einer Gegenelektrode verbunden wird. Die Haare können dann aufgrund ihrer Verbindung zu dem Körper selbst die Gegenelektrode für die durch die Zinken gebildete aktive Elektrode der Hochspannungsquelle bilden. Dabei ist die Gegenelektrode vorzugsweise auf Massepotential gelegt.

Bevorzugt ist jedoch eine Anordnung, bei der die Zinken einzeln abwechselnd oder in Gruppen abwechselnd mit zwei verschiedenen Anschlüssen einer Hochspannungsquelle verbunden werden, sodass sich unterschiedliche Potentiale zwischen benachbarten Zinken ergeben. Das zwischen den Zinken abgeteilte Haarbüschel wird dabei einer zwischen den Zinken ablaufenden Plasmabehandlung unterworfen. Durch die Zinken des Kamms werden parallele Haarsträhnen gebildet, die somit beim Durchziehen der kammähnlichen Struktur durch die Haare gleichzeitig und parallel der Plasmabehandlung unterworfen werden.

Ein Gerät zur Durchführung des Verfahrens nach der oben erwähnten ersten Ausführungsform ist erfindungsgemäß dadurch gekennzeichnet, dass es mit einem zangenförmigen Gerätekörper mit zwei Hebeln ausgebildet ist, mit denen eine abgeteilte Strähne des Haares erfassbar und zwischen einander gegenüberliegenden Anlageflächen der Hebel führbar ist und dass die Anlageflächen durch jeweils ein Dielektrikum gebildet sind, das jeweils eine mit einer Hochspannungsquelle verbindbare und auf einem der Hebel angeordnete flächige Elektrode abdeckt. Dabei sind die Elektroden vorzugsweise in das Dielektrikum eingebettet, also allseitig von dem Dielektrikum umgeben und lediglich mit einer Stromführung aus dem Dielektrikum herausgeführt. Die Dielektrika bilden dabei Anlageflächen, die in einer Ausführungsform der Erfindung glatt ausgebildet sind, während sie in einer anderen Ausführungsform der Erfindung mit Erhebungen und Rillen versehen sind, um eine abgeteilte Strähne noch in Teilsträhnen in Längsrichtung der Hebel des zangenförmigen Gehäusekörpers zu unterteilen.

Ein Gerät zur Durchführung des Verfahrens nach der oben erwähnten zweiten Ausführungsform ist erfindungsgemäß dadurch gekennzeichnet, dass es einen kammähnlichen Gerätekörper mit parallel zueinander verlaufenden Zinken aufweist, dass die Zinken mit einem leitenden, allseitig mit einem Dielektrikum umgebenden Kern ausgebildet sind und dass die leitenden Kerne mit einer Hochspannungsquelle verbunden sind.

Dabei ist vorzugsweise das die Zinken umgebende Dielektrikum als zusammenhängende einstückige Schicht ausgebildet.

Erfindungsgemäß wird somit die Plasmabehandlung von Haaren durch das Durchziehen einer kammähnlichen Struktur durch die Haare oder durch das Durchziehen einer Haarsträhne zwischen Auflageflächen zweier Hebel eines zangenähnlichen Geräts, wie es beispielsweise für das Glätten von krausem Haar bekannt ist, vorgenommen. Die Plasmabehandlung kann daher mit für jeden Menschen vertrauten Bewegungen erfolgen, sodass keine neuen Fertigkeiten erlernt werden müssen, um die erfindungsgemäßen Geräte anzuwenden.

Die für die Plasmabehandlung verwendeten Frequenzen für die alternierende Hochspannung liegen üblicherweise zwischen 1 Hz und 100 MHz. Die Einwirkzeiten der Plasmabehandlung richten sich nach dem Einsatzgebiet und können von einigen Millisekunden über mehrere Minuten bis hin zu einigen Stunden betragen.

Die Hochspannungen können einen Scheitelwert zwischen 100 und 100.000 V aufweisen. Die angelegte Spannung kann sinusförmig oder pulsförmig (unipolar oder bipolar) ausgebildet werden. Es ist auch eine Plasmabehandlung mit einer Gleichspannung möglich. Selbstverständlich könne alle Spannungsformen miteinander kombiniert werden.

Die Elektroden, in Form der flächigen Elektroden des zangenförmigen Gerätekörpers oder in Form der leitenden Kerne der Zinken, bestehen aus elektrisch gut leitenden Materialien. Sofern die Hochspannung zwischen den Elektroden des Geräts angelegt wird, können die Elektroden aus identischem Material gebildet sein. Denkbar ist aber auch die Verwendung des Trägers der Haare als Gegenelektrode, wobei als Träger der Haare insbesondere auch der menschliche oder tierische Körper bzw. die Haut in Frage kommen.

Der Abstand der Zinken der kammartigen Struktur kann zwischen 0,1 bis 5 mm liegen. Die gleiche Größenordnung gilt für die Rillen, die sich auf der Oberfläche des Dielektrikums quer zur Längsrichtung der zangenähnlichen Struktur erstrecken.

Die Erfindung soll im Folgenden anhand von in der Zeichnung dargestellten Ausführungsbeispielen näher erläutert werden. Es zeigen:
- Figur 1: eine Seitenansicht eines Geräts in einer ersten Ausführungsform, das als Zange mit zwei Hebeln ausgebildet ist, die im geöffneten Zustand dargestellt sind, wobei die Zuleitung zu jeweils einer auf den beiden Hebeln angeordneten Elektroden eingezeichnet ist;
- Figur 2: eine perspektivische Darstellung des Geräts gemäß Figur 1;
- Figur 3: eine Seitenansicht des Geräts gemäß Figur 1 im geschlossenen Zustand;
- Figur 4: eine grafische Darstellung der Kurvenverläufe der den beiden Elektroden zugeführten Hochspannungen;
- Figur 5: eine zweite Ausführungsform eines erfindungsgemäßen Geräts, das dem Gerät gemäß Figur 1 entspricht, wobei lediglich die Elektroden anders angeschlossen sind;
- Figur 6: eine grafische Darstellung der den Elektroden zugeführten Spannungen;
- Figur 7: ein drittes Ausführungsbeispiel eines Geräts der in Figur 1 dargestellten Art, wobei jedoch zueinander zeigende Anlageflächen mit Querrillen versehen sind;
- Figur 8: eine Seitenansicht des Geräts gemäß Figur 7 im geöffneten Zustand;
- Figur 9: eine Seitenansicht des Geräts gemäß Figur 7 im geschlossenen Zustand;
- Figur 10: eine perspektivische Darstellung eines Geräts in einer prinzipiell anderen Ausführungsform mit einem Gerätekörper und parallel zueinander zeigenden kammähnlichen Zinken, wobei das Gerät mit Spannungsverläufen gemäß Figur 4 versorgt wird;
- Figur 11: ein Gerät gemäß Figur 10, das mit Spannungsverläufen gemäß Figur 6 betrieben wird;
- Figur 12: eine schematische Darstellung für eine bei allen Ausführungsformen mögliche Art der Verbindung des Geräts mit der Hochspannungsquelle.

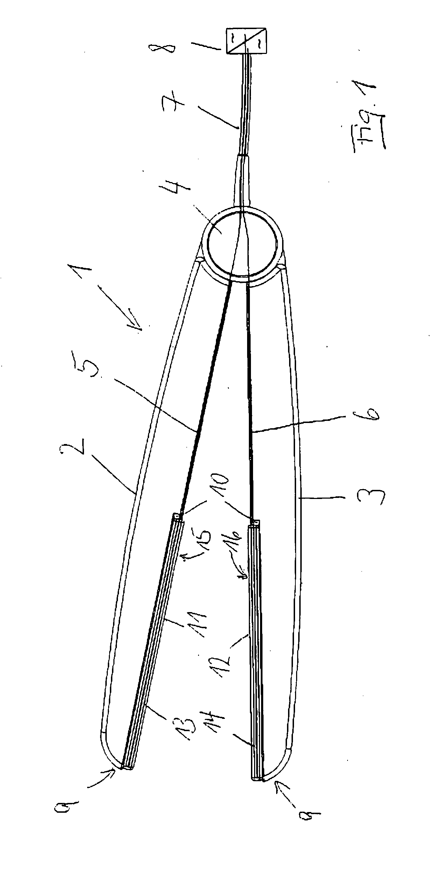

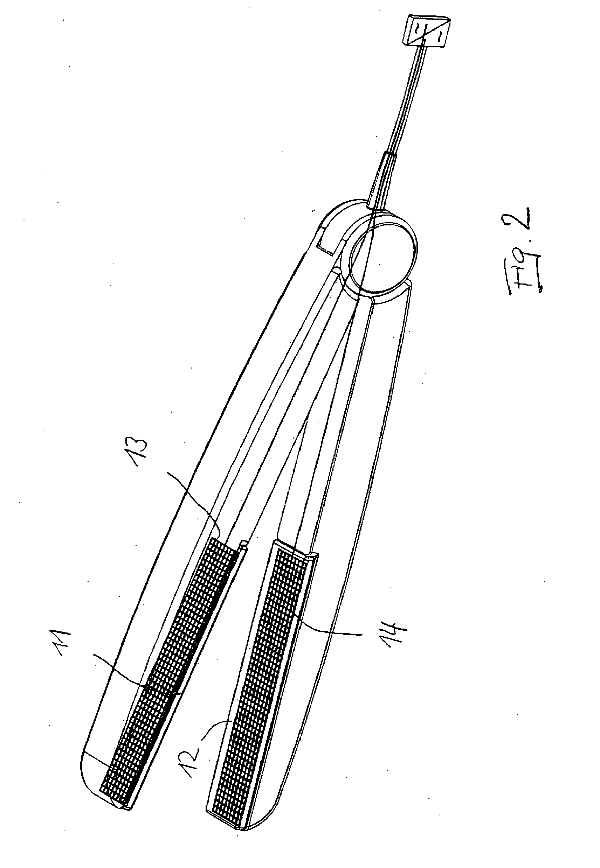

Gemäß den Figuren 1 bis 3 besteht ein Gerät in einer ersten Ausführungsform aus einem zangenförmigen Gerätekörper 1, der zwei einarmige Hebel 2, 3 aufweist, die an einer gemeinsamen Drehachse 4 gegeneinander schwenkbar verbunden sind, wobei die Öffnungsbewegung der beiden Hebel 2, 3 zueinander auf den in den Figuren 1 und 2 dargestellten Öffnungswinkel begrenzt ist. Die beiden Hebel 2, 3 sind als hohle Gehäuseteile ausgebildet, die in ihrem Innern eine Stromführung 5, 6 beinhalten, die mit einem Zuführungskabel 7 des Geräts verbunden sind. Das Zuführungskabel 7 ist an eine Hochspannungsquelle 8 angeschlossen.

Etwa über die Hälfte der Länge der Hebel 2, 3 sind diese - von ihren freien Enden 9 ausgehend - an den zueinander zeigenden Seiten mit jeweils einer Ausnehmung 10 versehen, in die jeweils ein Dielektrikum 11, 12 eingesetzt ist. Das Dielektrikum 11, 12 besteht aus einem nicht leitenden Material, vor-zugsweise aus Glas, Keramik oder Kunststoff mit dielektrischen Eigenschaften. In die beiden Dielektrika 11, 12 ist jeweils eine Elektrode 13, 14 eingebettet. Die Elektrode 13 ist mit der in demselben Hebel 2 verlaufenden Stromführung 5 verbunden, während die Elektrode 14 mit der Stromführung 6 verbunden ist. Da die Elektroden 13, 14 durch die Dielektrika 11, 12 von einander isoliert sind, wird ein Stromfluss zwischen den beiden Elektroden 13, 14 behindert.

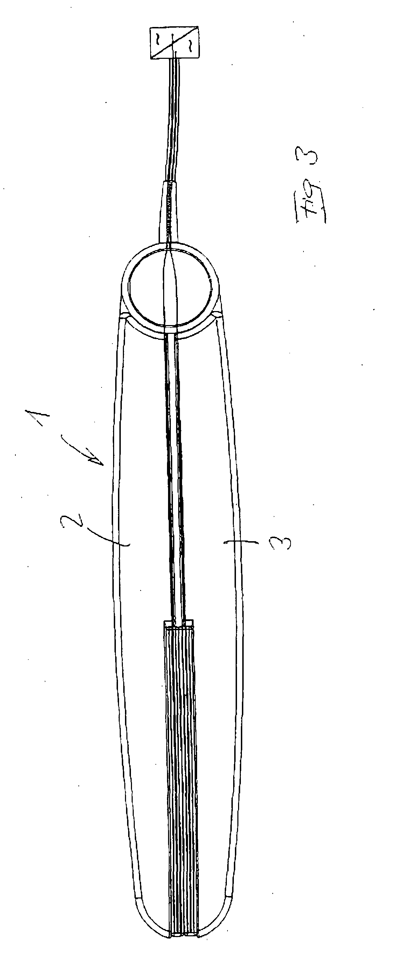

Die Dielektrika 11, 12 weisen auf den zueinander zeigenden Seiten ebene Anlageflächen 15, 16 auf, mit denen die Dielektrika im geschlossenen Zustand des zangenförmigen Gerätekörpers 1 (vgl. Figur 3) aneinander liegen. Die Anlageflächen stehen dabei aus den entsprechenden Oberflächen der Hebel 2, 3 außerhalb der Ausnehmung 10 etwas hervor, sodass die Anlageflächen 15, 16 beim Schließen des Gerätekörpers 1 unmittelbar zur Anlage kommen.

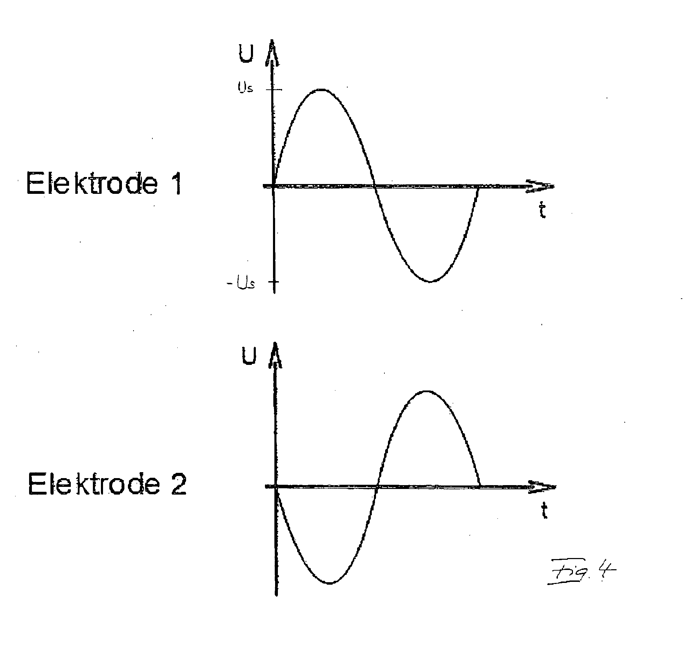

In der in den Figuren 1 bis 3 dargestellten Ausführungsformen sind beide Elektroden 13, 14 mit einem Wechselspannungsanschluss der Hochspannungsquelle 8 verbunden. Dabei sind jedoch die beiden Wechselspannungssignale um 180° zueinander phasenverschoben, wie dies in Figur 4 anhand von sinusförmigen Hochspannungssignalen mit einer Scheitelspannung Us dargestellt ist. Zwischen den Elektroden 13, 14 entsteht somit eine resultierende Wechselspannung mit einer Maximalspannung von 2 Us.

Die Scheitelspannungen Us können zwischen 100 und 100.000 V liegen. Die Spannung kann sinusförmig - wie in Figur 4 dargestellt, aber pulsförmig (unipolar oder bipolar) in Form von Hochfrequenzpulsen - ausgebildet sein.

Wie Figur 2 verdeutlicht, können die Elektroden 13, 14 in einer bevorzugten Ausführungsform als Gitter ausgebildet sein, was insbesondere beim Eingießen der Elektroden 13, 14 in einen thermoplastischen oder duroplastischen Kunststoff als Dielektrikum 11, 12 vorteilhaft ist, da sich der Kunststoff durch die Gitteröffnungen der Elektroden 13, 14 hindurch in flüssiger Form verteilen kann und somit für eine gute Einbettung der Elektroden 13, 14 in die Dielektrika 11, 12 sorgt.

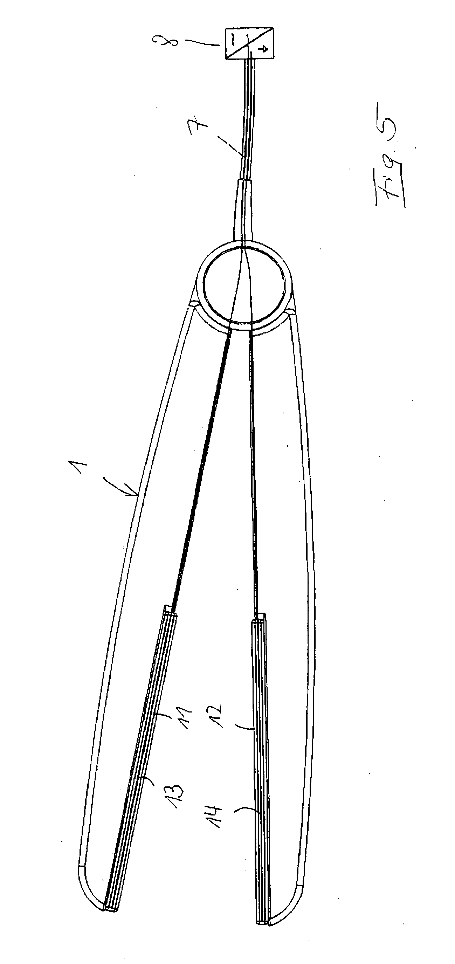

Das in Figur 5 dargestellte Ausführungsbeispiel stimmt bezüglich des Gerätekörpers 1 und der Ausbildung der Elektroden 13, 14 sowie der Dielektrika 11, 12 mit dem Ausführungsbeispiel gemäß den Figuren 1 bis 3 überein. Das Zuführungskabel ist hier jedoch an eine Hochspannungsquelle 8 derart angeschlossen, dass eine Elektrode 13 mit einer Wechselspannung verbunden ist, während die andere Elektrode 14 an Massepotential liegt. Somit ergibt sich zwischen den beiden Elektroden 13, 14 in diesem Fall ein Wechsel des Potentials mit der Maximalspannung Us bzw. -Us.

Im Gebrauch wird eine abgeteilte Strähne, beispielsweise des Kopfhaares eines Menschen, zwischen die Anlagefläche 15, 15 der Hebel 2, 3 gelegt und zwischen den gegeneinander gedrückten Hebeln 2, 3 gehalten. Durch einen Zug des Gerätekörpers 1 von der Kopfhaut der Person weg werden die Haare der Haarsträhne in Längsrichtung durch das Gerät hindurch gezogen, dessen Elektroden 13, 14 dabei mit den Anschlüssen der Hochspannungsquelle 8 verbunden sind. Auf diese Weise bildet sich zwischen den Dielektrika 11, 12 ein Plasma durch ionisierte Luft aus, mit dem die Haare zwischen den Dielektrika 11, 12 und den Elektroden 13, 14 behandelt werden.

Durch eine entsprechende Abtrennung zahlreicher Strähnen nacheinander kann beispielsweise das gesamte Kopfhaar einer Person behandelt werden. Es ist aber auch denkbar, das Haar lediglich in zueinander beanstandeten Strähnen zu behandeln, um beispielsweise eine strähnige Färbung des Haares vorzubereiten oder zu bewirken.

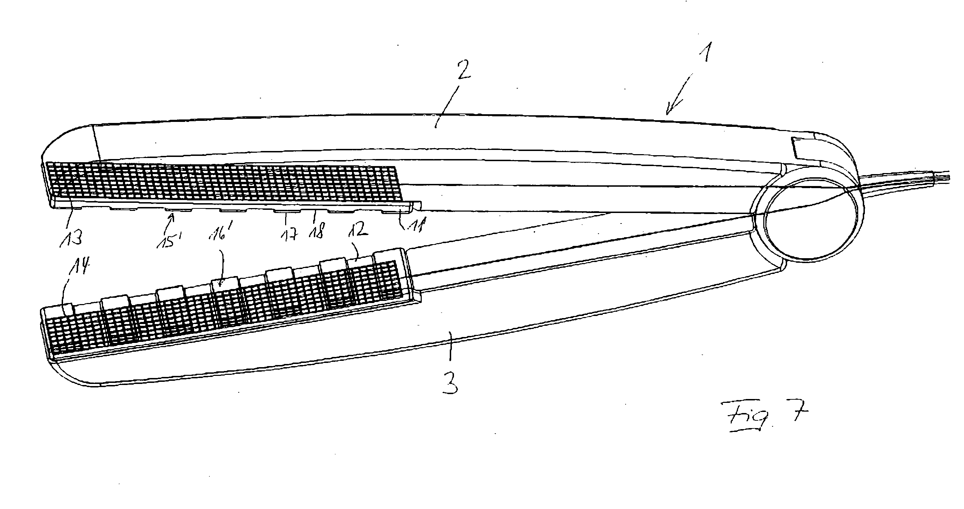

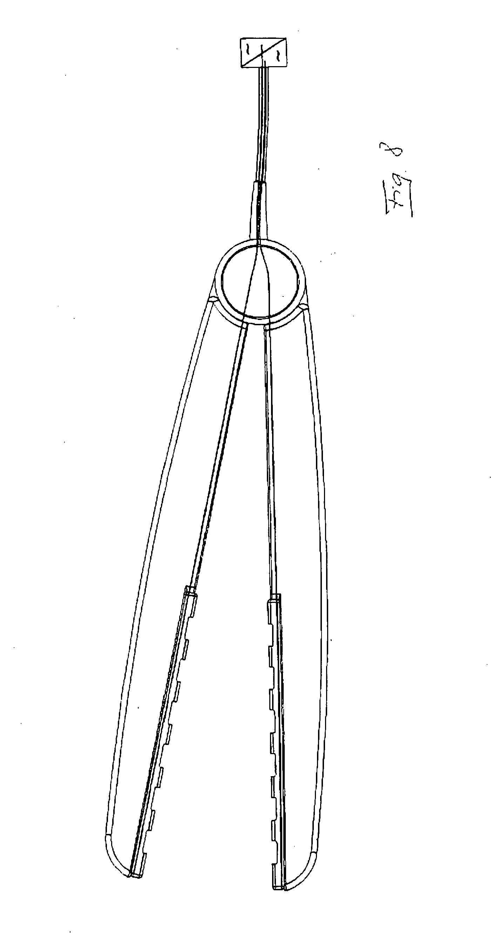

Bei der in den Figuren 7 bis 9 dargestellten weiteren Ausführungsform eines Behandlungsgeräts stimmen der Gerätekörper 1 und die Elektroden 13, 14 mit der Ausführungsform der Figuren 1 bis 3 bzw. der Figur 5 überein. Lediglich die Dielektrika 11, 12 weisen hier Anlageflächen 15', 16' auf, die durch abwechselnd angeordnete Vorsprünge 17 und Rillen 18 gebildet sind. Die Vorsprünge 17 und Rillen 18 verlaufen dabei quer zur Längsrichtung der Hebel 2, 3, sodass die Rillen 18 so ausgerichtet sind, dass beim Ziehen des Geräts von der Kopfhaut der Person weg die Haare durch die Rillen 18 gleiten können. Die Vorsprünge 17 können somit eine weitere Unterteilung der abgeteilten Haarsträhne bewirken und so die Plasmabehandlung intensivieren.

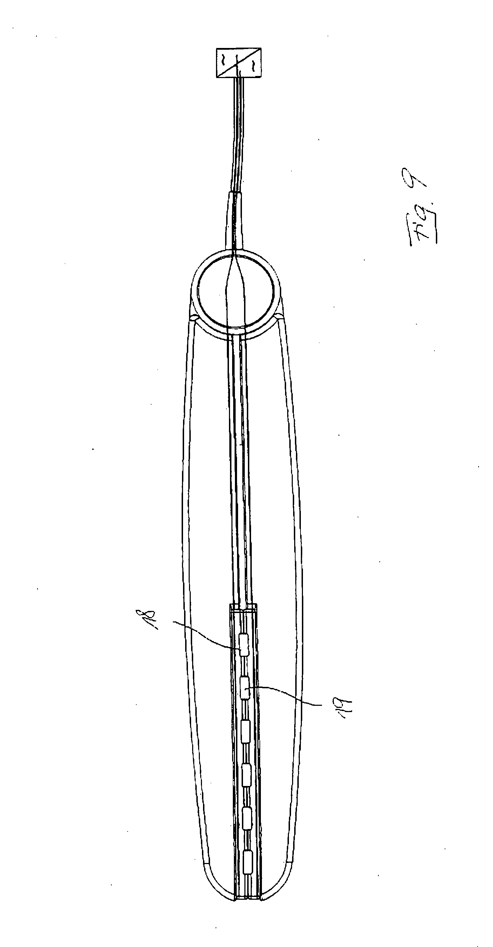

Figur 9 verdeutlicht, dass in dem geschlossenen Zustand des Geräts durch die Rillen 18 Queröffnungen 19 gebildet werden, durch die von den Strähnen abgeteilte Haarbüschel hindurchgezogen werden können.

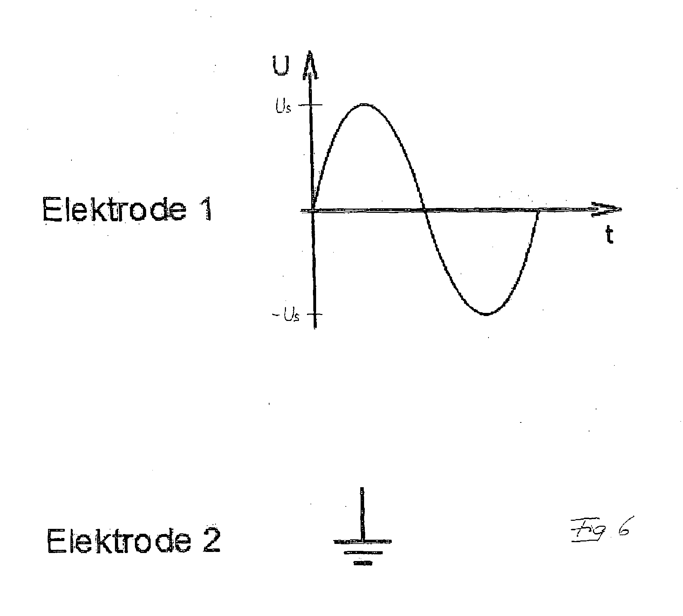

Es ist ohne weiteres ersichtlich, dass auch das Gerät gemäß den Figuren 7 bis 9 an die Hochspannungsquelle 8 gemäß Figur 4 oder auch gemäß Figur 6 angeschlossen werden kann.

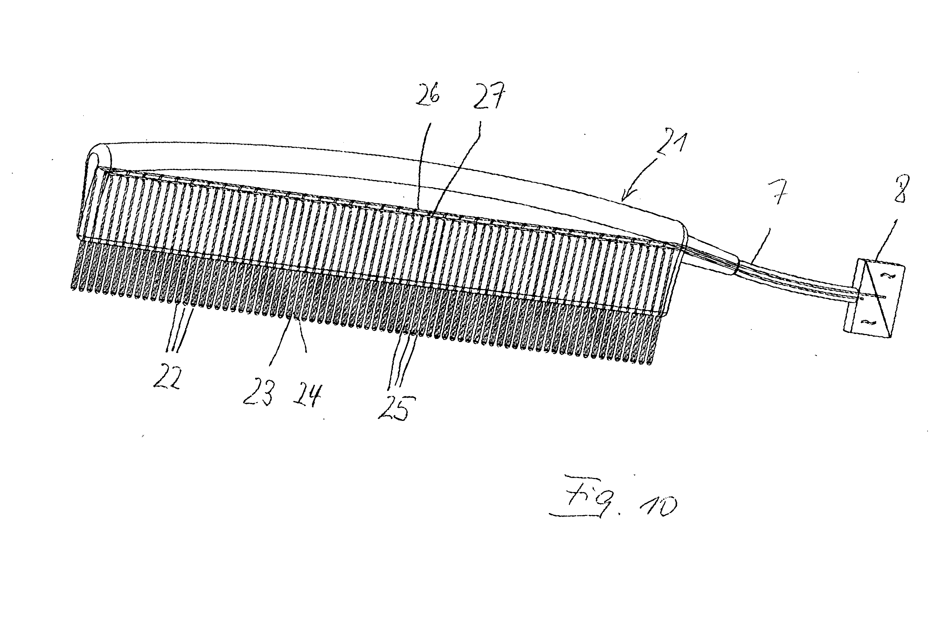

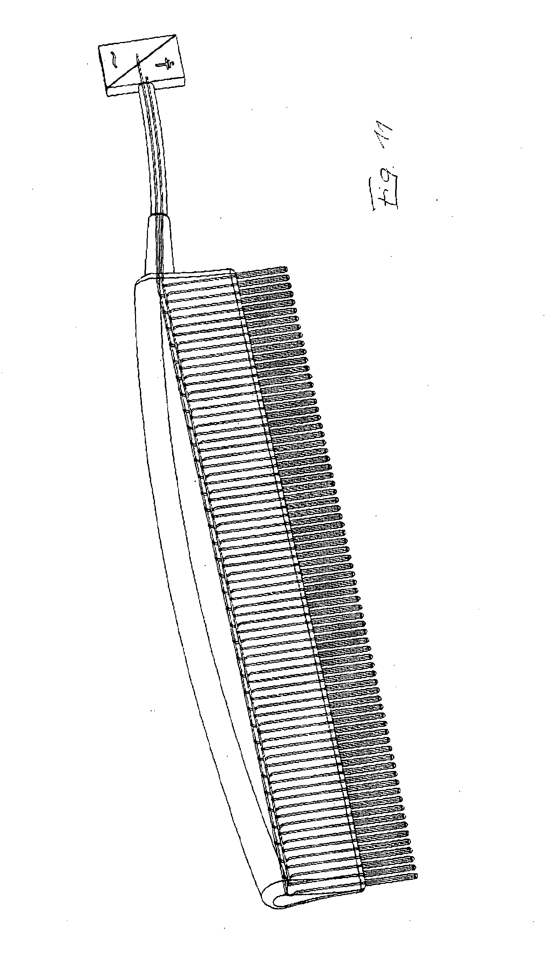

Bei dem in den Figuren 10 und 11 dargestellten Gerät führt das Zuführungskabel 7 der Hochspannungsquelle 8 in einen flachen, länglichen Gerätekörper 21, der an seiner Längsseite zahlreiche Zinken 22 trägt, die parallel zueinander verlaufen und aus dem Gerätekörper 21 um jeweils ein vorbestimmtes Maß herausragen. In dem in den Figuren 4 und 5 dargestellten Ausführungsbeispiel ragen die Zinken 22 alle mit einer gleichen Länge aus dem Gerätekörper 21 heraus. Da die Kante des Gerätekörpers 21, aus dem die Zinken 22 herausragen, geradlinig ist, bildet sich somit eine geradlinige Linie aus, auf der alle Zinken 22 enden.

Es ist aber auch möglich, mit den freien Enden der Zinken 22 eine gebogene und ggf. mehrfach gebogene Linie auszubilden, indem die Zinken 22 aus einer geraden Kante des Gerätekörpers 21 in unterschiedlichen Längen herausragen oder die Kante des Gerätekörpers 21 selbst entsprechend gekrümmt ausgebildet ist.

Die Zinken 22 bestehen jeweils aus einem aus dem Gerätekörper 21 herausragenden stiftförmigen leitenden Kern 23, der bis zum Gerätekörper 21 allseitig von einem Dielektrikum 24 umgeben ist. Zwischen den Zinken 22 sind Zwischenräume 25 ausgebildet, die einem gröberen Abstand üblicher Zinkenkämme entsprechen. Beim Ziehen des Geräts mit den Zinken 22 durch beispielsweise Kopfhaare eines Menschen werden diese in die Zwischenräume 25 zwischen den Zinken 22 in schmale Strähnen aufgeteilt.

In einer bevorzugten Ausführungsform befinden sich in dem Gerätekörper 21 zwei Stromführungen 26, 27, an die die Kerne 23 der Zinken 22 in zwei Gruppen jeweils parallel zueinander angeschlossen sind. In einer bevorzugten Ausführungsform ist an die Stromführung 26 jede zweite Zinke 22 angeschlossen, während an die Stromführung 27 die jeweils dazwischen liegenden Zinken 22 angeschlossen sind. Daraus ergibt sich, dass benachbarte Zinken 22 am unterschiedlichen Potential der Hochspannungsquelle 8 anliegen.

Es ist aber auch möglich, die Gruppen in anderer Weise geeignet auszubilden. Beispielsweise jeweils zwei nebeneinander liegende Zinken an dieselbe Stromführung 26 anzuschließen und links und rechts von diesen beiden Zinken 22 jeweils zwei Zinken mit der anderen Stromführung 27 zu verbinden. Denkbar ist auch jeweils zwei Zinken mit einem Potential und die jeweils benachbarten Zinken mit dem anderen Potential zu verbinden, sodass sich hintereinander eine Anordnung von 2-1-2-1... Zinken ergeben, wobei die jeweils zwei Zinken 22 mit der ersten Stromführung 26 und die jeweils einzelne Zinke 22 mit der Stromführung 27 verbunden ist. Weitere Kombinationen der Anschlüsse sind ebenfalls denkbar.

Die Kerne 23 der Zinken 22 bilden bei diesen Geräten die Elektroden, die jeweils von dem Dielektrikum 24 umgeben sind, sodass der Stromfluss zwischen den Elektroden 23 durch die Dielektrika 24 behindert wird.

Die Ausführungsbeispiele der Figuren 10 und 11 unterscheiden sich wiederum durch die Anschlüsse an die Hochspannungsquelle 8 gemäß Figur 4 (vgl. Figur 10) bzw. Figur 6 (vgl. Figur 11).

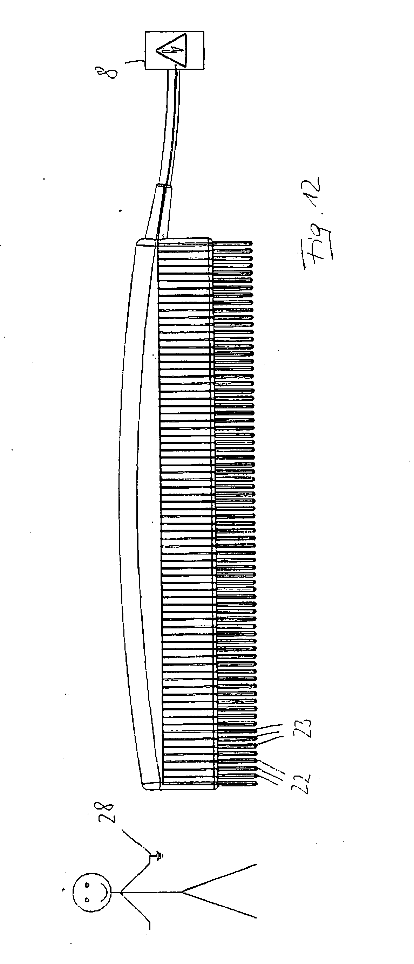

Figur 12 zeigt eine weitere Anschlussmöglichkeit für ein erfindungsgemäßes Gerät, die darin besteht, dass die die Elektroden bildenden Kerne 23 der Zinken 22 alle mit demselben Anschluss der Hochspannungsquelle 8 verbunden sind, vorzugsweise mit dem Wechselspannungsanschluss gemäß Figur 6. Zur Behandlung des Haares kann dabei der Träger des Haares, also beispielsweise ein Perückengewebe oder die das Haar tragende Haut eines Lebewesens mit einer Gegenelektrode 28 auf ein Referenzpotential gelegt werden, wobei das Referenzpotential vorzugsweise Masse ist.

In diesem Fall bildet der Träger des Haares bzw. das mit dem Träger verbundene Haar die Gegenelektrode 28 zu den Elektroden 23 des Behandlungsgeräts. Eine derartige Anordnung ist auch für ein zangenförmiges Behandlungsgerät gemäß den Figuren 1 bis 9 möglich und in bestimmten Anwendungsfällen mit Vorteil verwendbar.

Mit allen in der Zeichnung dargestellten Geräten lässt sich somit eine Plasmabehandlung der Oberfläche auf einem Träger fest angeordneten Haaren in einfacher Weise ausführen.

## Patentansprüche

1. Verfahren zur Behandlung von an einem Träger fest angeordnetem menschlichen oder tierischen Haar, **dadurch gekennzeichnet, dass** das Haar zu Strähnen aufgeteilt wird und dass die abgeteilten Strähnen durch ein Durchziehen eines mit einer Hochspannungsquelle (8) verbundenen Geräts durch die Strähne einer dielektrischen Plasmabehandlung unterworfen werden.

2. Verfahren nach Anspruch 1, **dadurch gekennzeichnet, dass** die abgeteilte Strähne zwischen zwei zangenförmig angeordneten flächigen Elektroden (13, 14) erfasst wird, die zur Strähne hin mittels jeweils eines Dielektrikums (11, 12) abgedeckt sind.

3. Verfahren nach Anspruch 1, **dadurch gekennzeichnet, dass** die Aufteilung des Haares in Strähnen mittels einer kammähnlichen Struktur mit parallel zueinander verlaufenden Zinken (22) erfolgt, dass die Zinken (22) mit einem leitenden, allseitig mit einem Dielektrikum (24) umgebenden Kern (23) ausgebildet sind und mit den leitenden Kernen (23) mit einer Hochspannungsquelle (8) verbunden werden.

4. Gerät zur Durchführung des Verfahrens nach Anspruch 1 oder 2, **dadurch gekennzeichnet, dass** es mit einem zangenförmigen Gerätekörper (1) mit zwei Hebeln (2, 3) ausgebildet ist, mit denen eine abgeteilte Strähne des Haares erfassbar und zwischen einander gegenüberliegenden Anlageflächen (16) der Hebel (2, 3) führbar ist und dass die Anlageflächen (16) durch jeweils ein Dielektrikum (11, 12) gebildet sind, das jeweils eine mit einer Hochspannungsquelle (8) verbindbare und auf einem der Hebel (2, 3) angeordnete flächige Elektrode (13, 14) abdeckt.

5. Gerät nach Anspruch 4, **dadurch gekennzeichnet, dass** die Elektroden (13, 1.4) in das zugehörige Dielektrikum (11, 12) eingebettet sind.

6. Gerät nach Anspruch 4 oder 5, **dadurch gekennzeichnet, dass** die Elektroden (13, 14) der beiden Hebel (2, 3) mit jeweils unterschiedlichen Spannungen der Hochspannungsquelle (8) versorgbar sind.

7. Gerät nach Anspruch 4 oder 5, **dadurch gekennzeichnet, dass** die Elektroden (13, 14) der beiden Hebel (2, 3) mit demselben Pol der Hochspannungsquelle (8) verbunden und mit identischen Spannungen versorgbar sind und dass eine Gegenelektrode (28) zur Verbindung mit dem elektrisch leitend ausgebildeten Träger des Haares vorgesehen ist.

8. Gerät zur Durchführung des Verfahrens nach Anspruch 1 oder 3, **dadurch gekennzeichnet, dass** es einen kammähnlichen Gerätekörper (21) mit parallel zueinander verlaufenden Zinken (22) aufweist, dass die Zinken (22) mit einem leitenden, allseitig mit einem Dielektrikum (24) umgebenden Kern (23) ausgebildet sind und dass die leitenden Kerne (23) mit einer Hochspannungsquelle (8) verbunden sind.

9. Gerät nach Anspruch 8, **dadurch gekennzeichnet, dass** das die Zinken (22) umgebende Dielektrikum (24) als zusammenhängende einstückige Schicht ausgebildet ist.

10. Gerät nach Anspruch 8 oder 9, **dadurch gekennzeichnet, dass** die Zinken (22) abwechselnd an unterschiedliche Spannungen der Hochspannungsquelle (8) zur Ausbildung eines Plasmas zwischen benachbarten Zinken (22) anschließbar sind.

11. Gerät nach Anspruch 8 oder 9, **dadurch gekennzeichnet, dass** die Zinken (22) an identische Spannungen der Hochspannungsquelle (8) anschließbar sind und dass eine Gegenelektrode (28) zur Verbindung mit dem Träger des Haars vorgesehen ist.

## Claims

1. A method for treating human or animal hair which is fixedly arranged on a support, **characterized in that** the hair is divided into strands, and **in that** the divided strands are subjected to dielectric plasma treatment by an apparatus which is connected to a high-voltage source (8) and which is drawn through the strands.

2. The method as claimed in claim 1, **characterized in that** the divided strands are grasped between two flat electrodes (13, 14) which are arranged in the form of tongs and which each are covered by means of a dielectric (11, 12) in the direction of the strands.

3. The method as claimed in claim 1, **characterized in that** the hair is divided into strands by means of a comb-like structure having tines (22) which run parallel to one another, **in that** the tines (22) are formed with a conductive core (23) which is surrounded on all sides by a dielectric (24) and are connected to a high-voltage source (8) by the conductive cores (23).

4. An apparatus for carrying out the method as claimed in claim 1 or 2, **characterized in that** said apparatus is formed with a tongs-like apparatus body (1) having two levers (2, 3) with which a divided strand of the hair can be grasped and can be guided between contact faces (16), which are situated opposite one another, of the levers (2, 3), and **in that** the contact faces (16) each are formed by a dielectric (11, 12) which in each case covers a flat electrode (13, 14) which can be connected to a high-voltage source (8) and is arranged on one of the levers (2, 3).

5. The apparatus as claimed in claim 4, **characterized in that** the electrodes (13, 14) are embedded in the associated dielectric (11, 12).

6. The apparatus as claimed in claim 4 or 5, **characterized in that** the electrodes (13, 14) of the two levers (2, 3) can each be supplied with different voltages of the high-voltage source (8).

7. The apparatus as claimed in claim 4 or 5, **characterized in that** the electrodes (13, 14) of the two levers (2, 3) are connected to the same pole of the high-voltage source (8) and can be supplied with identical voltages, and **in that** a counterelectrode (28) is provided for connection to the electrically conductive support of the hair.

8. The apparatus for carrying out the method as claimed in claim 1 or 3, **characterized in that** it has a comb-like apparatus body (21) having tines (22) which run parallel to one another, **in that** the tines (22) are formed with a conductive core (23) which is surrounded an all sides by a dielectric (24), and **in that** the conductive cores (23) are connected to a high-voltage source (8).

9. The apparatus as claimed in claim 8, **characterized in that** the dielectric (24) which surrounds the tines (22) is in the form of a cohesive integral layer.

10. The apparatus as claimed in claim 8 or 9, **characterized in that** the tines (22) can be connected alternately to different voltages of the high-voltage source (8) in order to form a plasma between adjacent tines (22).

11. The apparatus as claimed in claim 8 or 9, **characterized in that** the tines (22) can be connected to identical voltages of the high-voltage source (8), and **in that** a counterelectrode (28) is provided for connection to the support of the hair.

## Revendications

1. Procédé pour le traitement de cheveux humains ou de poils animaux agencés de manière fixe sur un support, **caractérisé en ce que** les cheveux ou les poils sont subdivisés en mèches, et **en ce que** les mèches séparées sont soumises à un traitement au plasma diélectrique en tirant les mèches à travers un appareil relié à une source à haute tension (8).

2. Procédé selon la revendication 1, **caractérisé en ce que** les mèches séparées sont prises entre deux électrodes planes (13, 14) agencées en forme de pince, qui sont recouvertes au moyen d'un diélectrique respectif (11, 12) en direction des mèches.

3. Procédé selon la revendication 1, **caractérisé en ce que** la subdivision des cheveux ou des poils en mèches a lieu au moyen d'une structure semblable à un peigne avec des dents (22) s'étendant parallèlement les unes aux autres, **en ce que** les dents (22) sont réalisées avec une âme conductrice (23) entourée sur tous les côtés avec un diélectrique (24), et sont reliées à une source à haute tension (8) avec les âmes conductrices (23).

4. Appareil pour la mise en oeuvre du procédé selon la revendication 1 ou 2, **caractérisé en ce qu'**il est réalisé avec un corps d'appareil (1) en forme de pince comprenant deux leviers (2, 3) au moyen desquels une mèche séparée des cheveux ou des poils peut être prise et peut être guidée entre des surfaces d'appui (16) opposées des leviers (2, 3), et **en ce que** les surfaces d'appui (16) sont formées respectivement par un diélectrique (11, 12) qui recouvre respectivement une électrode plane (13, 14) capable d'être reliée à une source à haute tension (8) et agencée sur l'un des leviers (2, 3).

5. Appareil selon la revendication 4, **caractérisé en ce que** les électrodes (13, 14) sont noyées dans le diélectrique associé (11, 12).

6. Appareil selon la revendication 4 ou 5, **caractérisé en ce que** les électrodes (13, 14) des deux leviers (3, 2, 3) peuvent être alimentées avec des tensions respectivement différentes de la source à haute tension (8).

7. Appareil selon la revendication 4 ou 5, **caractérisé en ce que** les électrodes (13, 14) des deux leviers (2, 3) sont reliées au même pôle de la source à haute tension (8) et sont susceptibles d'être alimentées avec des tensions identiques, et **en ce qu'**il est prévu une électrode antagoniste (23) pour la liaison avec le support, réalisé de manière à conduire l'électricité, des cheveux ou des poils.

8. Appareil pour la mise en oeuvre du procédé selon la revendication 1 ou 3, **caractérisé en ce qu'**il comprend un corps d'appareil (21) semblable à un peigne avec des dents (22) s'étendant parallèlement les unes aux autres, **en ce que** les dents (22) sont réalisées avec une âme conductrice (23) entourée sur tous les côtés avec un diélectrique (24), et **en ce que** les âmes conductrices (23) sont reliées à une source à haute tension (8).

9. Appareil selon la revendication 8, **caractérisé en ce que** le diélectrique (24) qui entoure les dents (22) est réalisé sous la forme d'une couche cohérente d'un seul tenant.

10. Appareil selon la revendication 8 ou 9, **caractérisé en ce que** les dents (22) sont susceptibles d'être branchées à des tensions différentes de la source à haute tension (8) pour la formation d'un plasma entre des dents voisines (22).

11. Appareil selon la revendication 8 ou 9, **caractérisé en ce que** les dents (22) sont susceptibles d'être branchées à des tensions identiques de la source à haute tension (8), et **en ce qu'**il est prévu une électrode antagoniste (28) pour la liaison avec le support des cheveux ou des poils.
